# EUROPEAN PATENT APPLICATION

(11) **EP 2 623 117 A1**
(43) Date of publication of application: **07.08.2013**
(21) Application number: 13165968.2
(22) Date of filing: 30.04.2010
(51) Int. Cl.: A61K 39/21

(54) **Immunogenic nanomedicine composition and preparation and uses thereof**

(30) Priority: 30.04.2009 HU 0900269; 14.05.2009 HU 0900298; 14.05.2009 HU 0900299; 14.05.2009 HU 0900300
(62) Divisional of application: 10719414.4
(71) Applicant: Genetic Immunity Kft., 1027 Budapest (HU)
(72) Inventor: Lisziewicz, Julianna, 1121 Budapest (HU); Töke, Enikö, 1089 Budapest (HU); Lörincz, Orsolya, 1047 Budapest (HU); Somogyi, Eszter, 2363 Felsöpakony (HU); Pandur, József, 2030 Érd (HU)
(74) Representative: Lengyel, Zsolt

(57) **Abstract**

The invention belongs to the field of immunotherapy and vaccines. More particularly, the invention relates to a pharmaceutical composition comprising nanoparticles being composed of macromolecules and linear polyethylenimine (1-PEI) or 1-PEI derivative, and a pharmaceutically acceptable solvent containing a sugar alcohol, as well as a method for the preparation thereof. Uses of said compositions are also provided.

## Description

The invention belongs to the field of immunotherapy and vaccines. More particularly, the invention relates to a pharmaceutical composition comprising nanoparticles being composed of macromolecules and linear polyethyleneimine (1-PEI) or 1-PEI derivative in a pharmaceutically acceptable liquid formulation, as well as a method for the preparation thereof. Uses of said compositions are also provided.

The development of carriers for the delivery of macromolecules, including plasmid DNA, RNA and oligonucleotides, has seen considerable progress in the last three decades. Since naked plasmid DNA is unstable under *in vivo* conditions due to its rapid degradation by nucleases, carriers are used to protect DNA or RNA from degradation, to facilitate uptake, to target specific cells, and to transfer DNA or RNA into the nucleus or cytoplasm (Aigner et al., 2002; Aigner, 2007; Höbel et al., 2008). Apart from viral vectors, non-viral gene delivery systems have recently received increasing interest since the use of viral vectors raised safety, efficacy and manufacturing concerns. Viral vectors have been developed to facilitate the delivery of therapeutic genes and immunogenic antigens because viruses evolved to efficiently enter into the cells, to overcome the cellular degradations and to express their genetic materials (Lungwitz et al., 2005; Gore, 2003). However, viral vector-based vaccines present significant manufacturing and safety challenges compared to plasmid DNA, and their repeated administration is restricted due to vector immunity (Lehrman, 1999; Gore, 2003).

Cationic polymers are particularly attractive for the delivery of nucleic acids, since they allow synthetic modification of their structure, enabling the enhancement of transfection efficiency and reduction in toxicity. The interaction between the positively charged polymer backbone and negatively charged DNA leads to the spontaneous formation of nano-size complexes (polyplexes, nanoparticles) in aqueous milieu. The compact structure of the polyplex core prevents the access of nucleases and other degradation factors to the enclosed nucleic acid drugs. The polyplexes should maintain their stability before cellular uptake by endocytosis and should escape from the endosomal compartment inside the cell.

Among the polycations presently used in gene delivery, polyethylenimine (PEI) takes a prominent position due to its potential for endosomal escape (Boussif, 1995). PEI and derivatives of PEI are in the central position of the non-viral vector based gene delivery literature reviews (Neu et al., 2005; Lungwitz et al., 2005) and patents (US Patent 6,013,240; PCT/US2005/039779; US2001/0031498A1).

PEI-s are synthetized in either a branched or linear morphological structure with completely different physico-chemical characters and manufacturing methods. The branched form of PEI (b-PEI; CAS/RN: 9002-98-6; synonyms: aziridine homopolymer, ethyleneimine homopolymer) can be synthesized by acid-catalysed polymerization of aziridine monomers but technically the overall reaction is hardly controlable and dangerous (von Harpe et al, 2000). The other drawback in using this compound in biologicals and *in vivo* experiments is that the monomer (ethyleneimine or aziridine) is a cytotoxic and carcinogen material (RTECS, Toxline). It is very reactive, reacts with all kind of nucleophiles (amines, thiols, alcohols, carboxylic acids, etc.) to the ring-opened product. It readily cross-links the DNA which is the basis for its carcinogenicity. Branched PEI has a high density and variety of amine groups (primary, secondary and tertiary) which have different reactivity in physiological environment. Due to this fact and to the branched morphology, in the non-viral gene delivery studies the branched form of PEI is used at a higher N/P ratio (10-50) in the condensation reaction of pDNA into nanoparticles - which is cytotoxic - than in case of linear PEI (Jeong et al, 2007).

Linear PEI (1-PEI; CAS/RN: 26913-06-04; synonym: polyamine) can be obtained by cationic ring opening polymerization of 2-substituted 2-oxazoline monomers, followed by acid-catalysed hydrolysis of the N-substituted polymer. Oxazoline is a cyclic 5 member ring with much less crowding and much less reactive than aziridine. It will form ring-opened products only with strong nucleophiles (like primary amines) and its reaction can be modified to allow different lengths of PEI chains. Oxazolines aren't usually considered mutagenic and neither is 1-PEI (Goddard et al, 1989; Zalipski et al, 1996). Neither one gives a positive Ames test. There are no enzymatic or chemical breakdown products from PEI that are reactive. Linear PEI contains only secondary amines (less reactive than primary amines) which are mostly protonated under physiological conditions.

The majority of the studies have on their central focus improving the efficacy and safety of new biodegradable polymers. It has been shown that the relative gene expression can be strongly influenced by the particle size, which, in turn, can be moderated by the molecular weight of the PEI derivative, the method of particle preparation, and the N/P ratio. The polyplex size decreases with increasing molecular weight, an excess of the polyamine and in low ionic strength media, most likely due to improved DNA compaction (Ogris et al., 1998). These polyplexes are most commonly formulated in either glucose solutions or physiological salt. The physiological salt solution, due to its high ionic strength, induces the aggregation of the polyplexes, thus decreasing their cellular uptake. After intravenal injection, the aggregation of these polyplexes represents a safety challenge for these formulations.

Targeted gene delivery capitalizes on the presence of specific cell surface receptors for DNA uptake into cells by receptor mediated endocytosis. Therefore receptor-binding ligands, like sugars, are coupled to polycationic compounds like PEI that bind and condense DNA and effectively enhance gene transfer efficiency (US2001/0031498A1; Diebold et al., 1999).

The suitability of linear PEI for gene transfer was investigated and compared to random copolymers of poly(2-ethyl-2-oxazoline)-co-poly(ethylenimine) and N-alkylethylenimine (Jeong et al., 2001; Brissault et al., 2003), or biodegradable, cross-linked cationic multi-block copolymers of PEI with esters, amides (PCT/US2005/039779), or PEG molecules (US 6,652,886). However, the large majority of copolymers used produce a shielding effect that counteracts effective DNA complexation, requiring higher molecular weight of PEI, increased N/P ratio, or cross-linking of the DNA-condensing polymer (Lungwitz et al., 2005).

As plasmid DNA-based vaccination and immunotherapy in humans begins to approach pharmaceutical reality, it is clear that a number of issues need to be comprehensively addressed, beyond the efficiency and safety of delivery systems.

One major problem when using polymeric carrier like PEI is that the complexes often suffer from poor physical stability leading to decomposition, loss of transfection efficiency and/or the tendency to aggregate, and thus require the preparation of fresh polyplexes directly prior to administration (Neu et al., 2005). Much research has already been done concerning the control of the size of the complexes and the prevention of the aggregation. In most of the studies, the attempts to control the aggregation behavior of polycations have either involved the right choice of environment and complexing protocols or covalent attachment of protecting groups (Ikonen et al., 2008). Recently, it was proposed that the aggregation could also be prevented by surfactants. Sharma et al. (2005) showed that the addition of the surfactant polyoxyethylene inhibits the aggregation process of PEI-DNA complex during 24-hour storage. Longer storage periods were achieved by liophylization of PEI-DNA / RNA complexes formed with low molecular branched PEI, nevertheless the linear PEI based complexes have completely lost their biological activity upon freezing (Höbel et al., 2008). The storage in a non-frozen state (4°C or room temperature) of the same complex led to extensive aggregation and complete inactivation of the complexes within 2 days (Höbel et al., 2008).

There is urgent need in the art to provide means for nucleic acid and antigen delivery that is pharmaceutically acceptable, protects the macromolecule from degradation and is biologically and chemically stable in selected storage temperature.

To provide a solution for the above challenges the present inventors surprisingly found a pharmaceutically acceptable liquid formulation suitable for stable complexation of antigens (a peptide, protein, plasmid DNA) with linear polyethylenimine (1-PEI) or 1-PEI derivative in a well-defined ratio. In particular, the present solution provides compacted nanoparticles with a diameter within the range from about 50 nm to about 500 nm, which provides protection for the antigens from degradations and supports their cellular delivery and its presentation to the host's immune system. The compact nanoparticles according to the present invention are termed herein as "NanoComp".

WO96/02655 describes compositions containing cationic polymers, e.g. PEI for the delivery nucleic acids into cells. Several factors that were deemed to be important for the preservation of biological activity of the nucleic acid were investigated. Complexes of PEI with nucleic acids are claimed at a ratio between 0,5 and 50, more preferably between 5 and 30, with the size of PEI in the range of 50 kDa and 800 kDa. Only complexes of the nucleic acids and cationic polymers are disclosed, formation of nanoparticles are neither taught nor suggested. Accordingly, the particular relevance of the nanoparticle formation for the biological efficiency, especially relevant for antigen delivery, was not revealed.

US2003/0027784A1 describes compositions containing low molecular weight PEI of 5 kDa to 50 kDa. These polymers are synthesized by polymerization of aziridine monomer which leads to the formation of branched three-dimensional PEI molecules. However, the described molecular weight range overlaps with molecular weight of the PEI polymers claimed in present invention, there is major difference in the structure of polymers. As described above, the topology of the PEI polymers is an important factor for their safety and efficiency during *in vitro* or *in vivo* use. Furthermore, it was shown that complexes formed between 1-PEI and DNA have different physico-chemical behavior from b-PEI/DNA complexes (Jeong et al, 2007).

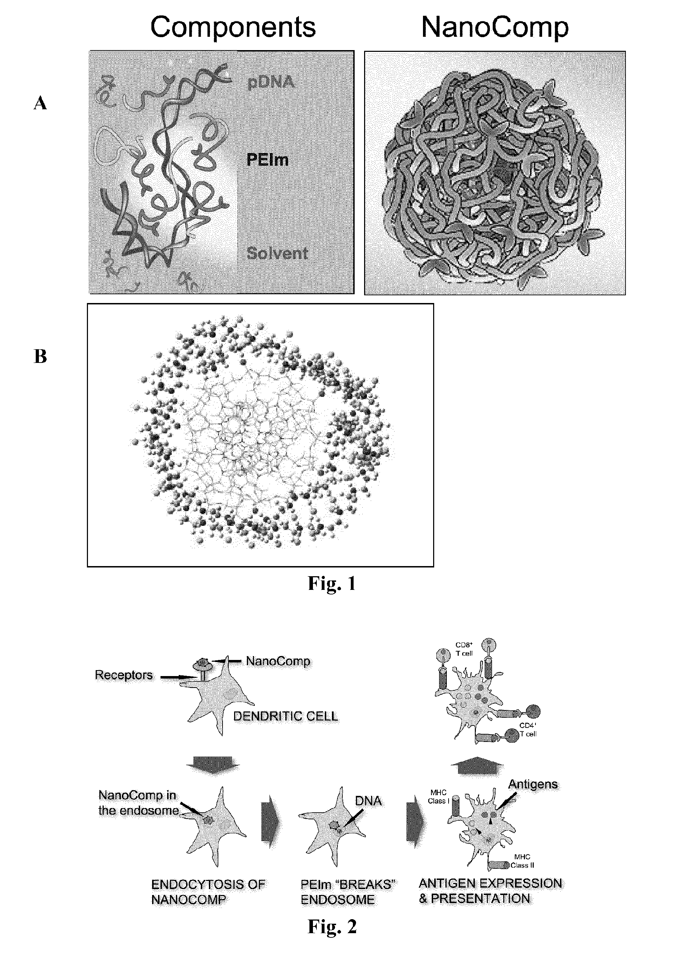

The imunogenic nanomedicine composition according to the present invention comprises pathogen-like intelligent nanoparticles generated from antigens (e.g. DNA, proteins, peptides) to mimic a pathogen exposure. A NanoComp resembles pathogens because it has condensed antigens in the core that enveloped with a polymer with sugar residues on the surface **(****Fig. 1A-B).** The NanoComp enters into antigen-presenting cells similarly to pathogens, primarily to the endosomes via receptor-mediated endocytosis **(****Fig. 2****).** Inside the cell, the polymer envelop of the NanoComp protects the antigen core from endosomal degradation and support antigen presentation. Importantly for pharmaceutical use, the NanoComp formulation improves the stability and immunogenicity of the antigens incorporated.

The NanoComp plasmid DNA formulation according the invention combines the advantages of viral vector-based and plasmid DNA vaccines. It utilizes chemical excipients to form a pathogen-like nanoparticle with features similar to viruses. PEIm (mannobiosylated polymer) is a novel excipient in the nanoformulation that not only protects the macromolecules from degradation but also facilitates the cellular uptake and transfer of the antigens into the cytoplasm and nucleus of the cells required for efficient antigen expression. NanoComp and its ingredients (macromulecule and excipients) can be manufactured consistently at large scale and administered repeatedly since it only contains formulation excipients beside the disease-specific antigens. The NanoComp formulation is particularly attractive for the delivery of nucleic acids due its excellent safety features and transfection efficiency, and can be used to induce immune responses for the prevention and treatment of diseases (e.g. chronic infectious pathogens, including intracellular viruses and bacteria, and neoplastic diseases, allergy).

Accordingly, the present invention provides a pharmaceutical composition comprising nanoparticles being composed of macromolecules and linear polyethylenimine (1-PEI) or 1-PEI derivative in a pharmaceutically acceptable liquid or lyophilized formulation, wherein the diameter of said nanoparticles is within the range from about 50 nm to about 500 nm, and wherein the molar ratio of the PEI or PEI derivative and the macromolecule is from about 2 to about 10, preferably from about 2 to about 6, and more preferably about 3 to about 4.5.

In a preferred embodiment said 1-PEI or 1-PEI-derivative is in a protonated salt form, wherein the ratio of the protonable N atoms / total N atoms is from about 0.1 to about 0.8, preferably about 0.4.

In another preferred embodiment, said 1-PEI or 1-PEI-derivative has a molecular weight less than 50kDa, preferably between 1kDa and 35kDa, more preferably between 10kDa and 25kDa.

In further preferred embodiment the macromolecule is a nucleic acid, preferably plasmid DNA or RNA, and preferably said nucleic acid encodes one or more antigens, more preferably is a virus-like particle (VLP). In a specific embodiment, the size of said nucleic acid is from about 3 kbp to about 20 kbp, preferably from about 6 kbp to about 13 kbp.

In another preferred embodiment the macromolecule is a polypeptide, and preferably the size of said polypeptide is from about 1 kDa to about 400 kDa, preferably from about 20 kDa to about 100 kDa.

In further preferred embodiments the liquid formulation of the pharmaceutical composition according to the invention is an aqueous solution having a pH value from about 5 to about 10, preferably from about 7 to about 8.

In another preferred embodiment the ionic strength of said formulation is from about 0.2 mM to about 50 mM, preferably from about 2 mM to about 5 mM.

In other preferred embodiments said pharmaceutically acceptable liquid formulation further comprises at least one sugar alcohol according to the general formula (I):

H(HCHO)nH (I)

wherein n is an integer from 2 to 12,
and wherein said sugar alcohols include all enantiomers and epimers.

In a specific embodiment, two, three or more different sugar alcohols are used.

In a further specific embodiment, n is 6, and said sugar alcohol is preferably selected from the group consisting of mannitol, sorbitol, xylitol and combinations thereof, more preferably said sugar alcohol is D/L-mannitol.

In further preferred embodiments the concentration of said sugar alcohols is in the range from 0 to about 50% (w/v), preferably from about 10% to about 30%.

In another preferred embodiment the pharmaceutical composition according to the invention is stable at 0-40 °C for a time period of at least 4 weeks, more preferably at least 8 weeks.

In a specific embodiment the the relevant biological activity of the macromolecule is maintained for a time period of at least 4 weeks, more preferably at least 8 weeks.

In another specific embodiment said macromolecule is a plasmid DNA, and wherein the physico-chemical properties (characterizable by the *oc*/*ccc* ratio) of said plasmid DNA are maintained at 0-40 °C for a time period of at least 1 week, more preferably at least 4 weeks, thereby said plasmid DNA is protected from degradation.

In another aspect, the invention provides a pharmaceutical composition, for use in inducing immune responses.

In a preferred embodiment, the pharmaceutical composition is provided for prevention or for treatment of chronic diseases.

In another preferred embodiment, the pharmaceutical composition is provided for use as a pharmaceutical to be introduced by needle injection or by mucosal, oral, vaginal, rectal or transdermal administration.

In another aspect, the present invention provides a method for the preparation of a pharmaceutical composition comprising nanoparticles being composed of macromolecules and linear PEI or PEI derivative, said method comprising mixing macromolecules with 1-PEI or 1-PEI derivative in a pharmaceutically acceptable solvent, wherein the molar ratio of the PEI or PEI derivative and the macromolecule is from about 2 to about 10, preferably from about 2 to about 6, and more preferably about 3 to about 4,5, thereby forming nanoparticles with a diameter from about 50 nm to about 500 nm.

In a preferred method of the invention, said PEI or PEI-derivative is in a protonated salt form, wherein the ratio of the protonable N atoms / total N atoms is from about 0.1 to about 0.8, preferably about 0.4.

In another preferred embodiment, said 1-PEI or 1-PEI-derivative has a molecular weight less than 50kDa, preferably between 1kDa and 35kDa, more preferably between 10kDa and 25kDa.

In another preferred embodiment, said macromolecule is a nucleic acid, preferably plasmid DNA or RNA, and preferably said nucleic acid encodes one or more antigens, more preferably is a virus-like particle (VLP). In a specific embodiment, the size of said nucleic acid is from about 3 kbp to about 20 kbp, preferably from about 6 kb to about 13 kbp.

In another preferred embodiment the macromolecule is a polypeptide, and preferably the size of said polypeptide is from about 1 kDa to about 400 kDa, preferably from about 20 kDa to about 100 kDa.

In further preferred embodiments the liquid formulation of the pharmaceutical composition according to the invention is an aqueous solution having a pH value from about 5 to about 10, preferably from about 7 to about 8.

In another preferred embodiment the ionic strength of said pharmaceutical composition is from about 0.2 mM to about 50 mM, preferably from about 2 mM to about 5 mM.

In other preferred embodiments said pharmaceutically acceptable liquid formulation further comprises at least one sugar alcohol according to the general formula (I):

H(HCHO)nH (I)

wherein n is an integer from 2 to 12,
and said sugar alcohols include all enantiomers and epimers.

In a specific embodiment, two, three or more different sugar alcohols are used.

In a further specific embodiment, n is 6, and said sugar alcohol is preferably selected from the group consisting of mannitol, sorbitol, xylitol and combinations thereof, more preferably said sugar alcohol is D/L-mannitol.

In further preferred embodiments the concentration of said sugar alcohols is in the range from 0 to about 50%, preferably from about 10% to about 30%.

In another embodiment, the ratio of the volume of solvent and the volume of macromolecule solution is from 0 to about 20, preferably about 6.

In a further embodiment, said sugar alcohols are added in a solution prepared in a pH buffer (6.5 < pH < 8.1), with a weaker base than the polymer, having the general formula (II):

NRR'R" (II)

wherein
if R=R'=R", then R is selected independently from C1-4 alkyl, C1-4 hydroxy-alkyl, C1 5 alkyloxy-C1-4 alkyl;
if R=phenyl; R',R" is selected independently from C1-4 alkyl, C1-4 hydroxyl-alkyl, C1 5 alkyloxy-C1-4 alkyl.

In a specific embodiment the base according to general formula (II) is preferably triethylamine or triethanolamine.

In another embodiment said pharmaceutical composition is reconstituted from a lyophilized state extemporaneously with sterile water.

In another embodiment said pharmaceutical composition is reconstituted from frozen state after thawing.

In another embodiment said macromolecule and said linear PEI or linear PEI derivative are separately frozen in buffered solution, and mixed together extemporaneously after thawing.

In another embodiment said macromolecule is reconstituted from a lyophilized state extemporaneously with a buffered linear PEI or linear PEI derivative solution.

As it was already discussed above PEI has been widely used for non-viral transfection *in vitro* and *in vivo* and has an advantage over other polycationes in that it combines strong DNA compaction capacity whith an intrinsic endosomolytic activity (Lungwitz et al., 2005).

### Detailed description

The present invention provides a pharmaceutical composition, comprising nanoparticles ("NanoComp") formed by an antigen (peptide, polypeptide, protein, DNA, RNA) and a linear PEI (1-PEI) or 1-PEI derivative in a pharmaceutically acceptable liquid formulation, which is stable at 0-40 °C for at least one month.

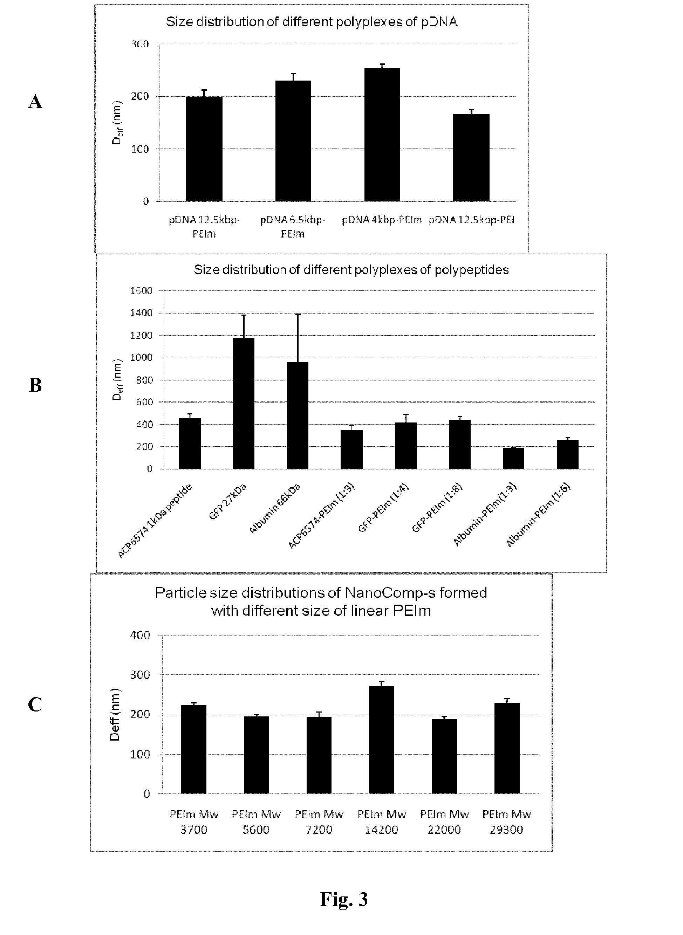

The antigen, polyanionic in a selected solvent, and the partially protonated, polycationic 1-PEI form by electrostatic interaction nanoparticles with effective diameter (D_{eff}) between 50 and 500 nm, preferably between about 100 and about 300 nm. The formation of stable nanoparticles require a slight excess of the 1-PEI (calculated as N concentration) added to the antigen, above a minimum molar ratio of about 2 of polycation added to antigen. The particle size of the nanoparticles is generally not affected by the excess of the polycation. The size of the formed nanoparticles, within the given range, is generally independent from the size of the antigen or 1-PEI derivative used **(****Fig. 3A - C).** Accordingly, in preferred embodiments of the present invention, the 1-PEI derivative formulated in nanoparticles can be a 1-PEI or 1-PEI derivative with molecular weight less than 50 kDa, preferably between 1kda and 35 kDa, more preferably between 10kDa and 25 kDa **(****Fig. 3C****).** Therefore, the statements and examples presented for the plasmid DNA (pDNA) and the linear, 3% mannobiose grafted PEI (which may be used interchangeably within the description with PEIm or other 1-PEI-derivatives, in particular in the context of nanoparticles formation) during the description are applicable for any antigen and 1-PEI or 1-PEI derivative. The formation of nanoparticles is proven by the fact that after complexation of the macromolecule (antigen) by the polymer, the size of the free antigen is considerably reduced resulting in a discrete particle size **(****Fig. 3B****).** For the polymer, having a linear topology, can not be measured any value for effective diameter by dynamic light scattering method (DLS), nor for the plasmids.

The antigen used in the nanoparticles according to the invention may be either a polynucleotide or a polypeptide.

As used herein, the term "polynucleotide" includes all kinds of nucleic acids irrespectively of origin, type, composition, substitutions, etc. For example, the nucleic acid may be both DNA and RNA, it may be synthetic or isolated from living organisms, constructed to include genes or markers of interest, may comprise irregular nucleotides, etc. Accordingly, in preferred embodiments of the present invention, the antigen formulated in nanoparticles can be a nucleic acid (DNA or RNA) with 3-20 kbp length, more preferably between 6 and 13 kbp **(****Fig. 3A****).**

The term "polypeptide" according to the present invention means molecules composed of any number of amino acids. Accordingly, the term includes shorter peptides of about 6-15 amino acids, up to long proteins of even 400 kDa in molecular weight. The polypeptide of the invention may comprise any kind of amino acids, may be synthetic or isolated from nature, and may have specific biological activity or serve as an immunogen. In specific embodiments of the invention, the antigen formulated in nanoparticles can be a polypeptide with a size of about 1 to about 400 kDa, preferably between 20-100 kDa.

The majority of the polypeptides in aqueous solution have their isoelectric points below our working pH (pH 6-8), therefore the proteins have polyanionic character which can also form nanoparticle complexes with the polycationic PEIm. There is evidence in the literature for the complex formation of PEI with fluorescently labeled avidin protein (Molecular Probes), and their transmembrane delivery into human fibroblasts and glial cells (Didenko et al., 2005). The nanoparticle characteristic of this complex was not studied. We show evidence for the first time that it is possible to formulate proteins into compact nanoparticles **(****Fig. 3B****).**

The NanoComps according to the present invention are antigen-containing mannosylated nanoparticles with a size in the range of about a few hundred nanometers, in aqueous solution resembling the size and appearance of viruses. The nucleic acids and/or other antigens are condensed in the core **(****Fig. 1A-B)** and on the positively charged polymer surface they may wear mannobiose residues to enhance their receptor-mediated uptake **(****Fig. 2****).** To strengthen its antigenic behavior, the NanoComp of the present invention may also wear alternative sugar residues on the surface and may also incorporate adjuvants (e.g. cytokines) to improve or redirect immune responses. These nanoparticles maintain their stability during storage before cellular uptake and escape from the endosomal compartment inside the cell. The NanoComp of the present invention provides a solution to the formulation, manufacturing and regulatory challenges of biologic and nanomedicine pharmaceuticals.

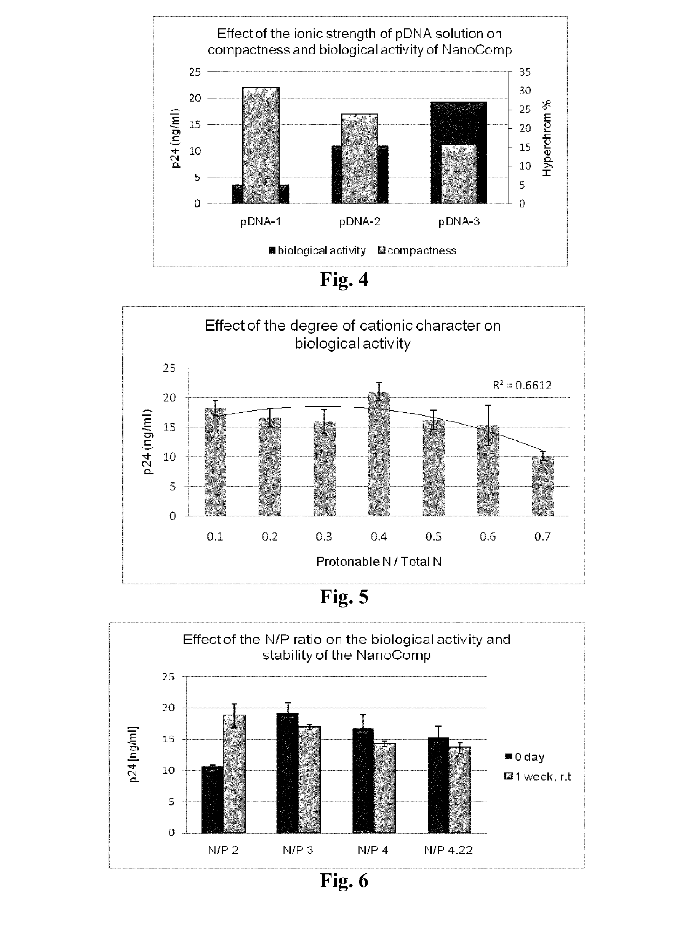

The present invention not only gives evidence that the structure (degree of association, compactness) of the nanoparticles is also a key parameter for the efficient gene delivery, but found out the tool for measuring it (see Example 5). The compactness (degree of association) of the nanoparticles is given by the type and number of interactions between the polyanionic pDNA and polycationic PEI(m), which can be measured spectrophotometrically as the absorbance enhancement at 260 nm of the nanoparticles compared to the absorbance of pDNA at same concentrations. It was revealed that the too high (or too low) number of interactions between the cationic polymer and the anionic antigen results in compact nanoparticles which are not favored by the gene delivery mechanism (Han et al., 2009), and because of the increased hydrophobicity of the particles their stability in aqueous solution is lowered. The number of interactions is given by the number of cationic secondary amines and the number of the anionic phosphates, which are predefined in certain conditions by the type and the physico-chemical characteristics of the raw materials **(****Fig. 4****).**

The cationic degree of the polymer has major effect, acting as a "proton-sponge" against the low pH of the endosome (Boussif, 1995). The cationic character of the polymer has an optimum value regarding the efficiency of the NanoComp which means that other efficiency parameter(s) require the opposite degree of the cationic character. Once localized inside the cells, in the cytosol, the polyplexes move to a proximal position of nucleus, and unload the pDNA. For this step of the delivery the compactness of the polyplex should be intermediate between the loose and very stable structure, which protects the pDNA from the too early and too late escape from the polycationic core. In this context, again, the degree of polycationic character of the PEIm and the anionic character of the pDNA (given by the pH and ionic strength of the solution) should be carefully selected. Accordingly, the ionic strength of the formulation according to the invention is between about 1 mM and about 50 mM, preferably between 2 and 5 mM. This also applies to the ionic strength of the antigen (macromolecule) solution.

In the context of the present description, the term "about", as used with reference to numerical values, means a deviation of ±15% from said value. The term "about", as used with reference to pH, refers to ±0.2 pH value.

The very low number of protonated (cationic) nitrogen atoms, which is preferable for endosomal escape, can make less number of ionic interaction with the more or less number of anionic phosphate of pDNA (depends on the ionic strength of the pDNA solution), and the complex formed has a loose structure, which is not stable enough in the cytosol or in the liquid formulation and cannot protect the pDNA. These processes require PEIm with an optimum degree of cationic character and pDNA solution with not too high ionic strength **(****Fig. 4** and **Fig. 5****).**

In addition to the ionic strength of the solution, the cationic character of the polymer may be preferably influenced by the selection of said polymer, In fact, the commercial PEI preparation (Polyplus-transfection, France) used in the present examples has a molecular weight of 25 kDa, accompanied with a ratio of the protonable N / Total N of about 0.1. The results presented herein ascertain that this ratio is not optimal for the optimal biological activity of NanoComps, the preferred range is being from about 0.1 to about 0.8, preferably about 0.4. As shown in Example 6, further decreasing the cationic character of PEI resulted in better characteristics for the nanoparticles **(****Fig. 5****).**

The molar ratio of PEIm to the macromolecules - such as pDNA, which gives the abbreviated name for this ratio, i.e. N/P ratio for nitrogen/phosphate ratio) or alternatively a protein or peptide - used for the construction of the nanoparticles should have the same effect on the biological activity of the NanoComp, making loose (N/P < 3) or compact structures. The effect of the N/P ratio on the biological activity of NanoComp prepared and tested freshly or after one-week storage in pH buffered solution is shown on **Fig. 6****.** A very good example of the importance of the relatively compact structure of the nanoparticles is shown in the case of the nanoparticles formed at N/P ratio of 2. Freshly prepared such nanoparticles had lower biological activity than after one-week storage of the NanoComp, or than the nanoparticles formed at higher N/P ratio. The reason for this behavior should be that at N/P ratio of 2 the freshly formed particles have not enough stable, compact structures, having effective diameter of 400 nm with very high SD (D_{eff} =339±133 nm) while after storage the particles became more compact (D_{eff}=280±7 nm). The average effective diameter of the nanoparticles formed at N/P >2 is 210±10 nm, independent on the N/P ratio and time of storage. The high N/P ratio may have toxic effect also for this reason the lowest N/P ratio of 3 was chosen for final formulations.

With respect to the biological activity of the macromolecule comprised in the NanoComp according to the present invention, the person skilled in the art will be able to determine which activity of the macromolecule is considered to be relevant for the purposes of the specific application. In preferred embodiments, this relevant biological activity is preserved or at least is not significantly decreased with the use of the NanoComp of the invention for elongated periods of time. It is also possible that the macromolecule has several quantifiable biological activities, and any of those may be maintained by the NanoComp. As long as at least one of these activities is the activity to be maintained, the NanoComp of the invention will fulfill its purpose as specified herein, but this is not limiting with respect to the preservation of any of the other activities of the macromolecule. In specific embodiments, where the macromolecule is a nucleic acid, this relevant biological activity can be its *oc*/*ccc* ratio, which characterizes its physico-chemical properties, and by maintaining this ratio at the original value it helps to keep the compact structure of the NanoComp substantially unchanged and thus protects the macromolecule (antigen) from degradation.

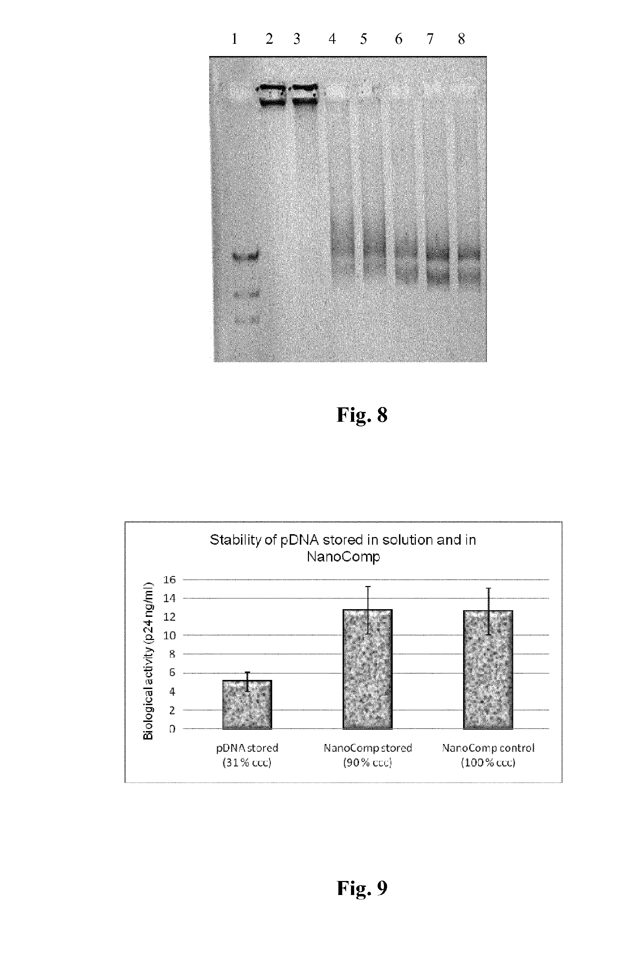

Making liquid formulation for these ionic particles, the pH of the solution has effect on the stability of the nanoparticles. The low pH of the solution can induce the aggregation and subsequent precipitation of the nanoparticles and also the degradation of the pDNA (the pharmaceutically active ingredient). The stability of the pDNA inside the NanoComp proved to be dependent on the pH of the formulation solvent, at higher, pH > 6.5 the active pharmaceutical ingredient (API) did not show degradation (**Fig. 8A**). The convenient NanoComp formulation (pH=7.5) totally protects the homogeneity and biological activity of the pDNA even at 37°C compared to the control (fresh) pDNA **(****Fig. 9****).**

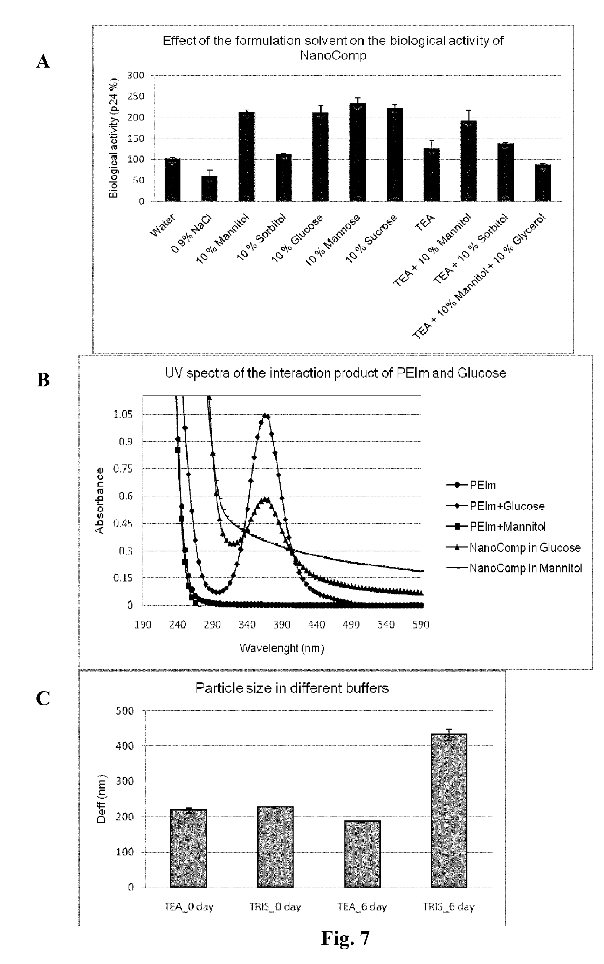

The type of the solvent used should further stabilize the nanoparticles, the sugar-like compounds have improving effect on the biological activity of the NanoComp compared to sterile water **(****Fig. 7A****).**

Sterile water and 10% glucose or dextrose solutions are commonly used and suggested as pharmaceutically acceptable solvents (US Patent 6,013,240), but none of them proved to be applicable for the formulation of polyplexes. Glucose was shown to have stabilizing effect on nanoparticles, but over time it interacts with the amino groups of the PEIm forming an adduct called Amadori's product which has Aₘₐₓ at 365 nm (yellow) (Bhattacharyya 1995; Tjan and Ouweland, 1974) **(****Fig.7B****).** For this reason reductive sugars, like glucose, mannose etc. could not be useful in stable formulations, but other sugar-like compounds, such as sugar-alcohols, like mannitol, proved to have the same improving effect on the biological activity, but without side reactions with PEIm **(****Fig. 7A-B).** Since the formulations with the aqueous solution of the sugars have pH only between 5 and 6, for stability concerns the sugar-alcohol solution is made in pharmaceutically acceptable pH buffer, which is weaker base than the secondary amine containing linear PEIm (to outpace the side reaction with the acidic pDNA). Thus, bases containing tertiary amines, like triethanolamine, are pharmaceutically approved, nonreactive substances for buffering nucleic acid based compositions, in contrast to primary amine containing Tris [tris(hydroxymethyl)aminomethane] buffer, which during time increases the particle size of the NanoComp **(****Fig. 7C****).** The pH of the buffers should be between 6.5 and 8.1. The lower pH was shown to have degrading effect on the pDNA **(Fig. 8A)**, at the higher pH the PEIm precipitates.

Since the detailed characterization of the starting materials proved to be of high importance in biological activity and stability of the formulation, the long-term stability studies were effectuated using two different lots of pDNA (with different ionic strength) and two different lots of PEIm (with different degree of cationic character) (Table 2).

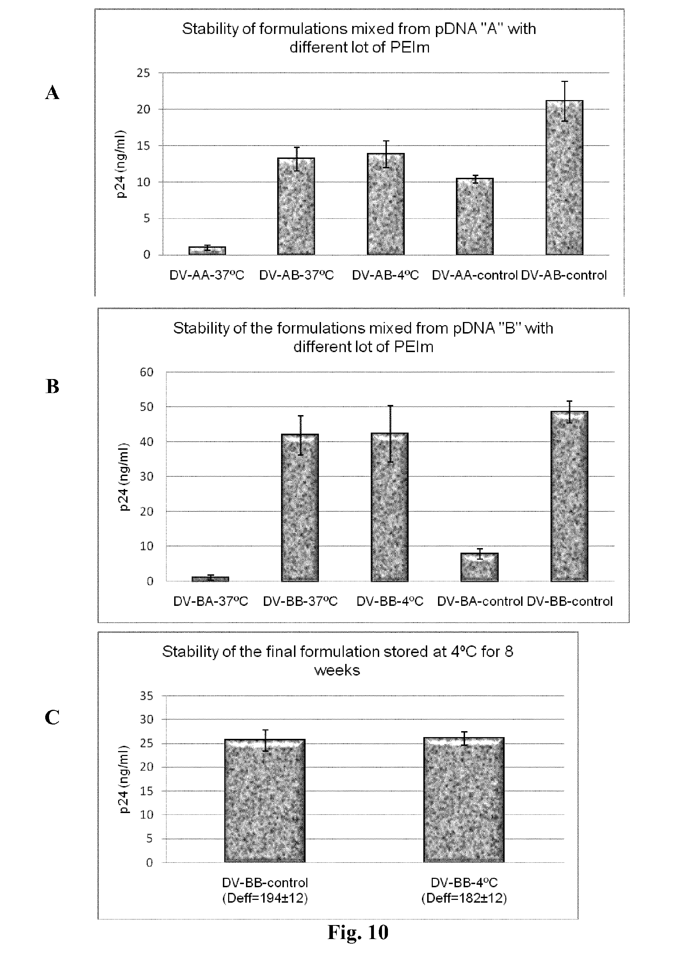

The biological activity of the NanoComp mixed using pDNA solution with high ionic strength (30mM, pDNA-A) and PEIm of high degree of cationic character (90% of the amine N were protonated, PEIm-A) stored at 37°C was much lower compared to the control than the potency of the NanoComp mixed with PEIm with low degree of cationic character (60% of the amine N were protonated, PEIm-B), at the same temperature and compared to its own control. However the potency of the NanoComp mixed with pDNA-A and PEIm-B stored at 4°C and 37°C was the same, their potency during 3 week storage dropped significantly compared to the control **(****Fig. 10A****).** The major event occurred during storage was the aggregation and precipitation of the nanoparticles, which explain the reduced biological activity.

The biological activity of the NanoComp formulations mixed using pDNA-B solution with low ionic strength (1.5 mM) and PEIm-A stored at 37°C was much lower compared to the control than the biological activity of the NanoComp mixed with PEIm-B at the same temperature and compared to its own control **(****Fig. 10B****).** The major event occurred during storage was the partial decomplexation of the nanoparticles (lowered hyperchromicity from 20% to 2%), which explain the partial degradation of pDNA inside the nanoparticle (61% *ccc* remained) and thus the reduced biological activity.

The biological activity of the NanoComp formulations mixed with pDNA-B and PEIm-B stored at 4°C and 37°C was the same, their potency during 3 week storage doesn't dropped significantly compared to the control formulation **(****Fig. 10B****).**

The best composition of tested NanoComp liquid formulation proved to be pDNA in low ionic strength solution, PEIm with low degree of cationic character, using at N/P ratio of 3 and formulated in triethanolamine buffered (pH 7.5) mannitol solution, which was stable at 4°C for 8 weeks without aggregation or decomplexation and without loss of biological activity **(****Fig. 10C****).**

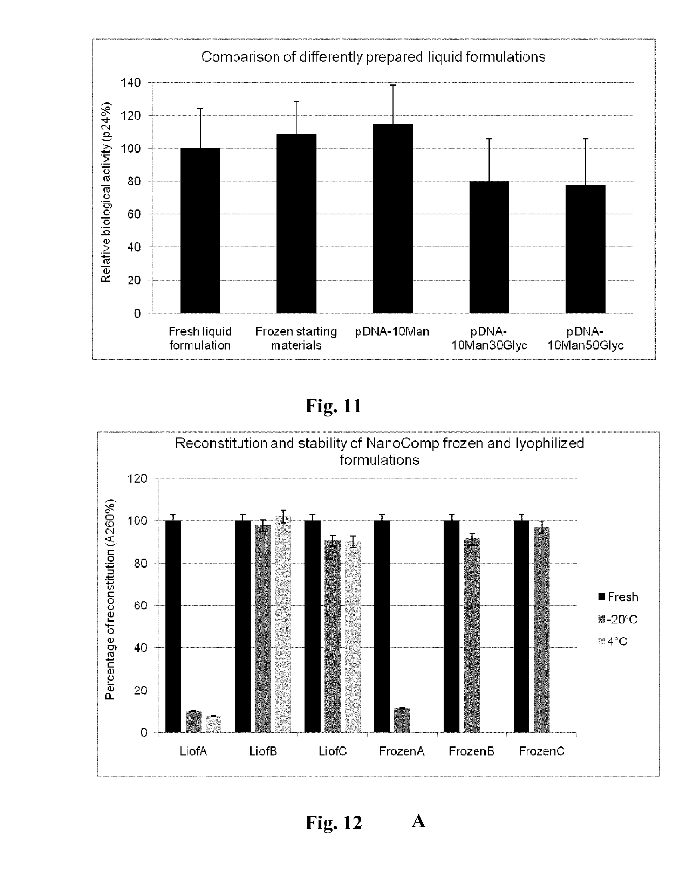

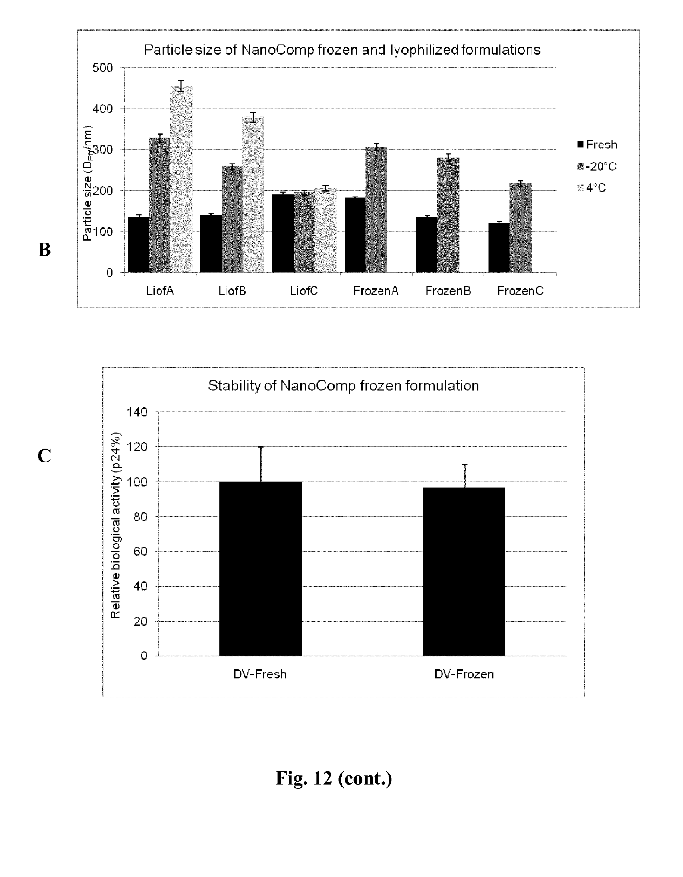

The composition complemented with glycerol allowed the storage of the NanoComp formulation in frozen or lyophilized state for 4 weeks. After reconstitution the biological activity of the stored NanoComp formulations doesn't dropped significantly compared to the control formulation **(****Fig. 12A-C).**

The composition according to the present invention proved to be more efficient than the compositions commonly used (e.g. in water or glucose solution). For manufacturing of a stable, pharmaceutically acceptable liquid formulation there are several possible pathways, such as the following non-limiting examples:
- preparation from liquid starting materials by mixing an aqueous solution of pDNA with 6 volume of triethanolamine-buffered mannitol and adding the PEIm aqueous solution (Example 3).
- preparation from frozen starting materials by storing the triethanolamine-buffered mannitol solution of pDNA at -20 °C and separately the triethanolamine buffered mannitol solution of PEIm at -20 °C, and mixing the two solutions together after thawing (Example 12).
- preparation from lyophilized starting materials by making lyophilized powder from the pDNA with mannitol and reconstituting the solution with a buffered PEIm solution (Example 13).
- Preparation of lyophilized formulation of the NanoComp and reconstitution with sterile water (Example 14).
- Preparation of liquid formulation of the NanoComp than frozen and reconstitution by allowing to defrost at room temperature (Example 15).

The person skilled in the art will be readily capable to determine the optimal set up for the preparation of the NanoComp, including the volumes, concentrations and types of starting materials.

As it was discussed above, the NanoComps according to the present invention are pathogen-like nanoparticles generated from antigens (e.g. DNA, proteins, peptides) to mimic a pathogen exposure. The most common routes by which antigens are introduced into the body are subcutaneous, intradermal, transdermal, intramuscular, intravenous or intraperitoneal administration. The pharmaceutical composition according to the present invention may be administered vaginally or rectally for the prevention of infectious diseases as microbicide. Oral administration provides antigens into the gastrointestinal tract, while intranasal administration or inhalation brings antigens into the respiratory tract. The preferred mode of administration according to the present invention is the use of the transdermal route.

We have demonstrated that pDNA/PEIm NanoComp formulation is not influenced by the sequence of the pDNA-encoded antigens or the size of the pDNA. Most of the above experiments, however, were performed with HIV pDNA.

The present invention is further illustrated by the experimental examples described below with reference to the figures, however, the scope of the invention will by no means be limited to the specific embodiments described in the examples. It is obvious, however, for the person skilled in the pertinent art that the claimed scope of the invention also encompasses other advantageous embodiments not exemplified herein but being possible to elaborate on the basis of the herein disclosed specific inventive concept and the general state of the art. The scope of the herein claimed invention is, therefore, to be considered as being exclusively limited by the appended claims.

### Description of the figures

**Fig. 1****.** Illustration of the nanoparticle formulation of antigens: A: NanoComp according to the invention. B: Molecular model of the NanoComp formulation calculated using Gaussian modeling method on 8 bp double stranded DNA at N/P ratio of 4.
**Fig. 2****.** Illustration of the pathogen-Iike mechanism of entry, antigen expression and antigen presentation with the NanoComp according to the invention.
**Fig. 3****.** Illustration of the particle size distribution of the NanoComp formulations prepared using different macromolecules and polymers: **A:** Formulations of 4, 6.5 and 12.5 kbp length pDNA-s with linear PEIm. **B:** Particle size of three polypeptides: ACP6574 1kda peptide, GFP 27kDa protein and Albumin 66 kDa protein without PEI; formulations of the polypeptides (1-66 kDa) with linear PEIm at molar ratios of 3, 4, 6 and 8. **C:** Formulations of 3, 5, 7, 14, 22 and 29 kDa linear PEIm with pDNA.
**Fig. 4****.** Illustration for the effect of the ionic strength of the pDNA solution on the compactness of the nanoparticles (measured as hyperchromicity) and on their biological activity.
**Fig. 5****.** Illustration for the effect of the cationic character (protonable N/Total N) of the PEIm on the biological activity of the nanoparticles.
**Fig. 6****.** Illustration for the effect of the N/P ratio on the biological activity and stability of the NanoComp stored in liquid formulation for 1 week at room temperature (approx. 25°C).
**Fig. 7****.** Illustration for the effect of the formulation solvent. **A:** Biological activity of the NanoComp formulations prepared with water, physiological saline, sugars, sugar alcohols, buffer (pH 7.5) and sugar alcohol in buffer (pH 7.5). **B:** Stability of the NanoComp formulations prepared with D-(+)-Glucose and D-(+)-Mannitol, with and without side reactions. **C:** Particle size of the NanoComp formulations prepared with triethanolamine buffer (pH 7.5) and Tris buffer (pH 7.5) and stored for 6 days at room temperature (approx. 25°C).
**Fig. 8****.** Illustration for the effect of the formulation pH on the pDNA stability (*oc*/*ccc* ratio) inside the NanoComp. **A:** Lanes: 1: DNA marker; 2: pH 3.1 (*ccc %:* degraded); 3: pH 3.8 (*ccc %:* degraded); 4: pH 5.3 *(ccc* %: degraded); 5: pH 5.9 *(ccc* %: degraded); 6: pH 6.8 *(ccc* %: 43.1); 7: pH 8.1 *(ccc* %: 43.3); 8: pDNA control + SDS *ccc* %: 45).
**Fig. 9****.** Illustration on the protecting effect of the NanoComp formulation on the pDNA homogeneity, when stored at room temperature (approx. 25°C) for 18 days.
**Fig. 10****.** Illustration on the stability of 4 NanoComp formulations prepared using 2 lots of pDNA and 2 lots of PEIm. **A:** Stability of the NanoComp formulations prepared from pDNA-A (in high ionic strength solution) with PEIm-A (high degree of cationic character) and PEIm-B (low degree of cationic character) stored for 3 weeks at 4 °C and 37 °C. **B:** Stability of the NanoComp formulations prepared from pDNA-B (in low ionic strength solution) with PEIm-A (high degree of cationic character) and PEIm-B (low degree of cationic character) stored for 3 weeks at 4 °C and 37 °C. **C:** Stability of the NanoComp final formulation prepared from pDNA-B (in low ionic strength solution) with PEIm-B (low degree of cationic character) stored for 8 weeks at 4 °C.
**Fig. 11**. Illustration on the manufacturing of NanoComp liquid formulation from frozen or lyophilized materials, stability and biological activity compared to the freshly prepared NanoComp liquid formulation.
**Fig.12****.** Illustration on the manufacturing of NanoComp frozen or lyophilized formulations. A: Reconstitution and stability of frozen and lyophilized NanoComp formulations. B: Particle size of frozen and lyophilized NanoComp formulations. C: Stability and biological activity of a representative frozen formulation compared to the freshly prepared NanoComp liquid formulation.

### Example 1: Description of the NanoComp formulations used in the experiments

### Preparation of the NanoComp formulations according to the present invention

The aqueous solution of plasmid DNA, pLWXu1 12.5kbp, 1 mg/ml solution encoding HIV-1 antigens (see Table 1) was diluted with 6 volume equivalent solvent and PEIm (linear polyethylenimine, 22 kDa grafted with 3% mannobiose, if not stated otherwise, 13.6 mM solution, Protonable N / Total N = 0.4) was added. The PEIm/pDNA complexes were prepared at different ratios of nitrogen/phosphate (N/P). The actual N/P ratio and the type of the solvent are labeled separately at every formulation.

**Table 1.**

| **General features of pDNA used** | **Specific features of DermaVir pDNA** |
|---|---|
| **HIV-specific antigen expression** | Gag, Protease, Reverse transcriptase, Env, Tat, Rev, Vif, Vpr |
| **Virus-like particle release** | Yes |
| **Safety features** | Integrase defective |
| | Reverse transcription impaired |
| | Nef truncated |
| **Regulation of gene expression** | HIV-1 LTR promoter |

### Biological activity assay:

The *in vitro* biological activity test of the NanoComp is performed by transfection of 293T human cells. 50,000 cells are plated in 96 well plate 19 hours before transfection, using DMEM culture medium (DMEM, 1% FBS, 1% L-glutamine, 1% Penicillin-Streptomycin). 0.5µg pDNA/NanoComp is added to each well followed by 21 hours incubation at 37°C in 5% CO₂ air condition. During the incubation period, the HIV-specific protein antigens encoded by the pDNA are expressed and secreted into the culture medium. After 21 hours, the supernatant of the cells are collected, and HIV-1 p24 antigen ELISA is performed to quantify p24 protein produced by the pDNA.

### Example 2: NanoComp formation using different antigens and polymer

a) Four NanoComp formulations were prepared according to Example 1 using PEIm for the formulation of different length of plasmid DNA (pLWXu1 12.5 kbp, pGL2 plasmid 6.5 kbp, pJET 1.2 plasmid containing 700 bp insert) at N/P ratio of 3 and with 10% D-Glucose solvent. The complexation of pLWXu1 plasmid was performed also with linear PEI (22 kDa) at N/P ratio of 3 in 10% D-Glucose solution. The particle size measurements were performed by dynamic light scattering method (DLS) using Brookhaven ZetaPals Instrument. Results are shown in **Fig. 3A****.**
b.) Five NanoComp formulations were prepared according to Example 1 using PEIm for the formulation of different size of polypeptides (ACP6574 standard, 1kda peptide; Green fluorescens protein (GFP), 27kDa protein, Millipore; and Albumin protein, 66 kDa, Sigma) at PEIm:polypeptide molar ratio of 3, 4, 6 or 8 and with triethanolamine-buffered (pH 7.5) mannitol solution. The size of the polypeptides without complexation is also measured. The particle size measurements were performed by DLS with Brookhaven ZetaPals Instrument. Results are shown in **Fig. 3****B.**
c.) Four NanoComp formulations were prepared according to Example 1 using different molecular weight of PEIm (linear polyethyleneimine containing 3% grafted mannobiose of Mn 3700, 5600, 7200, 14200, 22000 and 29300 g/mol determined by GPC, Genetic Immunity) for the formulation of plasmid DNA (pLWXu1 12.5 kbp), at N/P ratio of 3 and with 10% Mannitol solvent. The particle size measurements were performed by DLS using Brookhaven ZetaPals Instrument. Results are shown in **Fig. 3C****.**

### Example 3: Effect of the ionic strength of the pDNA solution

Three NanoComp formulations were prepared using three lots of pLWXu1 plasmid DNA. The formulations were prepared according to Example 1, using the same PEIm at N/P ratio of 4.22 and 10% D-Glucose as solvent. The supercoiled content *(ccc* form) of the plasmids determined by agarose gel electrophoresis was the same: 78±10%, but the ionic strength of the pDNA1, pDNA2 and pDNA3 plasmid solutions (determined by ICP-MS) were 1.5, 30 and 44mM, respectively. The absorbance measurements at 260 nm of the plasmid DNA solutions (1 mg/ml) and the prepared NanoComp formulations were performed in the same dilution using Jasco V-630 spectrophotometer. The calculated compactness was given by the percent increase (hyperchromicity) of the NanoComp absorbance at 260 nm compared to the pDNA solution (PEIm and the solvent have no Aₘₐₓ value at this wavelength). Results are shown in **Fig. 4****.**

### Example 4: Effect of the degree of cationic character of the PEIm

Different NanoComp formulations were made according to Example 1 using the same pDNA and PEIm containing different amount of protonable N, at N/P ratio of 3 and using Mannitol (triethanolamine buffered, pH 7.5) as solvent. The partially protonated PEIm were prepared starting from the PEIm (Polyplus Transfection SAS) containing 90% protonated N (Protonable N/ Total N = 0.1, by measuring the chlorine counterion by AOX) and proportionally neutralizing with 0.1 M NaOH solution to the required protonable N / Total N ratio. Results are shown in **Fig. 5****.**

### Example 5: Effect of the N/P ratio on the biological activity and stability of the NanoComp

Four NanoComp formulations were prepared according to Example 1, using the same PEIm at N/P ratios of 2, 3, 4, 4.22 and Mannitol (triethanolamine buffered, pH 7.5) as solvent. The effect of the N/P ratio on the biological activity was studied on the freshly prepared formulations, and after 1 week storage at room temperature (approx. 25°C). The particle size measurements were performed using Brookhaven ZetaPals Instrument. Results are shown in **Fig. 6****.**

### Example 6: Effect of the formulation solvent

a) NanoComp formulations were prepared according to Example 1, using the same PEIm and pDNA at N/P ratio of 3, and different solvents: sterile water, physiological NaCl (0.9%), D(+)-Mannitol (10 mg/ml), Sorbitol (10 mg/ml), D(+)-Glucose (10 mg/ml), Mannose (10 mg/ml), Sucrose (10 mg/ml), Triethanolamine buffer (TEA) (pH 7.5), D(+)-Mannitol (10 mg/ml) in triethanolamine (TEA) buffer (pH 7.5), Sorbitol (10 mg/ml) in triethanolamine buffer (pH 7.5) and D(+)-Mannitol (10 mg/ml) and Glycerol (10 mg/ml) in triethanolamine buffer (pH 7.5). The biological activity is given procentual related to the biological activity of the formulation in sterile water. Results are shown in **Fig. 7A****.**
b) PEIm solution, mixture of PEIm with Glucose (10%) and Mannitol (10%) respectively, NanoComp formulations with Glucose (10%) and Mannitol (10%) were prepared according to Example 1, the missing component in all mixtures were replaced with water, to ensure the same concentration of the components. The prepared solutions were stored at room temperature (approx. 25°C) for 2 month. UV spectra were recorded without dilution. Results are shown in **Fig. 7****B.**
c) NanoComp formulations were prepared according to Example 1, using the same PEIm and pDNA at N/P ratio of 3, and different buffers: Triethanolamine buffer 10 mM, pH=7.5 and Tris buffer 10 mM, pH 7.5. The particle size of the formed complexes was measured using ZetaPALS, Brookehaven instrument freshly and after 6 days storage at room temperature. Results are shown in **Fig. 7C****.**

### Example 7: Effect of the formulation pH on pDNA stability

NanoComp formulations were prepared according to Example 1, using the same PEIm and pDNA at N/P ratio of 3, and different solvents using D-Mannitol (10 mg/ml in buffer) prepared in the same buffer with different pH. The effect of the formulation pH was measured on the degradation of the pDNA inside the NanoComp during storage at room temperature for 18 days. The *oc*/*ccc* ratio of the pDNA inside the nanoparticles was measured by agarose gel electrophoresis (AGE) after decomplexation with 2% SDS solution (0.8% agarose gel, 100 V, 120 min running). Results are shown in **Fig. 8****.**

### Example 8: Stability of the pDNA inside the NanoComp formulation

NanoComp formulations were prepared according to Example 1, using the same PEIm and pDNA at N/P ratio of 3, using D(+)-Mannitol (10 mg/ml) in Triethanolamine buffer (pH 7.5) as solvent. The NanoComp formulations were stored for 1 week at 37°C. In the same time plasmid DNA (1 mg/ml aqueous solution) was also stored at the same temperature and time period. The biological activity of the pDNA in two forms, was checked by preparing NanoComp from the pDNA stored in aqueous solution and compared to the freshly prepared NanoComp. The stability (homogenity) of the pDNA in aqueous solution and formulated in NanoComp were checked by AGE after decomplexation with SDS using the same method as in Example 8, and is presented as the % *ccc* form remained compared to the fresh pDNA. Results are shown in **Fig. 9****.**

### Example 9: Stability of the NanoComp liquid formulations

Four NanoComp formulations were prepared according to Example 1, using two lots of pDNA and two lots of PEIm at N/P ratio of 3 and Mannitol (in Triethanolamine buffer, pH 7.5) as solvent. The specific parameters of the components and the abbreviation of the formulation prepared are listed in

**Table 2.**

| **NanoComp Formulation** | **pDNA-A** (ionic strength:30mM) | **pDNA-B** (ionic strength:1.5mM) | **PEIm-A** (Protonable N/Total N=0.1) | **PEIm-B** (Protonable N/Total N=0.4) |
|---|---|---|---|---|
| **DV-AA** | + | - | + | - |
| **DV-AB** | + | - | - | + |
| **DV-BA** | - | + | + | - |
| **DV-BB** | - | + | - | + |

The stability (measured in their biological activity compared to the freshly prepared control compositions) of the formulations mixed by the different starting materials was studied during three-weeks storage at 4°C and/or 37°C **(****Fig. 10A** and **Fig. 10B****).** The stability of the best composition was further investigated at 4°C for 8 weeks **(****Fig. 10C****).**

**Example 10: Preparation and stability of the NanoComp formulation prepared from frozen materials** NanoComp formulation was prepared from frozen starting materials. The pDNA solution mixed with 3 volume of triethanolamine buffered mannitol solution, and PEIm solution mixed with 3 volume of triethanolamine buffered mannitol solution were separately stored at -20 °C for 2 weeks. For the preparation of the NanoComp liquid formulation the two starting materials were allowed to defrost then the two solutions were mixed at N/P ratio of 3. The biological activity of the formulation was measured and compared to the freshly prepared liquid formulation according Example 1 **(****Fig. 11****).**

### Example 11: Preparation and stability of the NanoComp formulation prepared from lyophilized materials

NanoComp formulations were prepared from lyophilized pDNA. The pDNA solution was mixed with 6 volume of 10% mannitol solution (pDNA-10Man) or 10% mannitol+30% glycerol (pDNA-10Man30Glyc) or 10% Mannitol+50% Glycerol (pDNA-10Man50Glyc) in Triethanolamine buffer pH 7.5 and was lyophilized and stored for 30 days at 4 °C. For the preparation of the NanoComp liquid formulations the lyophilized pDNA powder was reconstituted with triethanolamine-buffered PEIm solution (pDNA-10Man) or PEIm aqueous solutions (pDNA-10Man30Glyc and pDNA-10Man50Glyc) respectively at N/P ratio of 3. The biological activity of the formulations was measured and compared to the freshly prepared liquid formulations according Example 1 **(****Fig. 11****).**

### Example 12: Preparation, reconstitution and stability of the NanoComp lyophilized formulations

NanoComp formulations were frozen at -20°C or lyophilized. The NanoComp formulations were prepared according to Example 1, using pDNA and PEIm at N/P ratio of 4 and 10% Mannitol (FrozenA and LyophA, respectively), 10% Mannitol + 30% Glycerol (FrozenB and LyophB, respectively) or 50% Glycerol (FrozenC and LyophC, respectively) in Triethanolamine buffer, pH 7.5 as solvent. The lyophilized formulations were stored for 30 days at -20°C and 4°C. For the preparation of the NanoComp liquid formulation, the lyophilized powders were reconstituted with water, the frozen formulations were defrosted. The degree of reconstitution of the frozen and lyophilized formulations was measured at 260 nm (Fig.12A) along with the particle sizes (Fig. 12B).

### Example 13: Biological activity and stability of the NanoComp frozen and refrigerated formulation

The biological activity of the Frozen C NanoComp formulation prepared in Example 14 was measured. This is a representative measurement for all frozen and lyophilized formulations prepared in Example 14 which could be 100 % reconstituted and their particle size were maintained. The biological activity of the formulation was measured and compared to the freshly prepared liquid formulation (Fig. 12B).

### References

A. Aigner; D. Fisher; T. Merdan; C. Brus; T. Kissel; F. Czubayko, Gene Ther., 9(24), 2002, 1700-1707.
A. Aigner, Curr. Opin. Mol. Ther., 9(4), 2007, 345-352.
S. Bhattacharyya, J. Org. Chem., 60(15), 1995, 4928-4929.
O. Boussif, Proc. Natl. Acad. Sci. USA., 92(16), 1995, 7297-301.
B. Brissault; A. Kichler; C.Guis; C. Leborgne; O. Danos; H. Cheradame, Bioconjugate Chem., 14, 2003, 581-587.
V.V. Didenko; H. Ngo; D.S. Baskin, Analytical Biochemistry, 344, 2005, 168-173.
S.S. Diebold; M. Kursa; E. Wagner; M. Cotten; M. Zenke, J. Biol. Chem., 274(27), 1999, 19087-19094.
P. Goddard; LE. Hutchinson; J. Brown; J. Brookman, J. Control Release, 10, 1989, 5-16. M.E. Gore, Gene Ther., 10, 2003, 4.
X. Han; Q. Fang; F. Yao; X. Wang; J. Wang; S. Yang; B.Q. Shen, Cytotechnology 60, 2009, 63-75.
A. von Harpe; H. Petersen; Y. Li; T. Kissel, J. Control. Release 69, 2000, 309-322.
S. Höbel; R. Prinz; A. Malek; B. Urban-Klein; J. Sitterberg; U. Bakowsky; F. Czubayko; A. Aigner, EJPB, 70, 2008, 29-41.
M. Ikonen; L. Murtomaki; K. Kontturi, Colloids and Surfaces B: Biointerfaces, 66, 2008, 77-83.
J.H. Jeong; S.H. Song; D.W. Lim; H. Lee; T.G. Park, J. Controll. Release, 73, 2001, 391-399.
J.H. Jeong; W.K. Sung; T.G Park, Progress in Polymer Science, 32, 2007, 1239-1274.
S. Lehrman, Nature, 401, 1999, 517-518.
U. Lungwitz; M.Breuning; T. Blunk; A. Göpferich, EJPB, 60, 2005, 247-266.
M. Neu; D. Fisher; T. Kissel, J. Gene Med., 7, 2005, 992-1009.
M. Ogris; P. Steinlein; M. Kursa; K. Mechtler; R. Kircheis; E. Wagner, Gene Ther. 5, 1998, 1425-1433.
V.K. Sharma; M. Thomas; A.M. Klibano, Biotechnol. Bioeng., 90, 2005, 614-620.
S. B. Tjan and G. A. M. V. D. Ouweland, Tetrahedron, 30(16), 1974, 2891-2897.
S. Zalipsky; CB. Hansen; IM. Oaks; TM. Allen, J Pharm Sci, 85, 1996, 133-137.

## Claims

1. Pharmaceutical composition comprising nanoparticles with a diameter within the range from 50 to 500 nm being composed of macromolecules and linear polyethylenimine (1-PEI) or 1-PEI derivative, and a pharmaceutically acceptable solvent containing a sugar alcohol.

2. The pharmaceutical composition according to claim 1, wherein said macromolecule is a nucleic acid and/or polypeptide in an aqueous solution having a pH value from 5 to 10, preferably from 7 to 8, and wherein the ionic strength of said pharmaceutically acceptable solvent is from 0.2 mM to 50 mM, preferably from 2 mM to 5 mM.

3. The pharmaceutical composition according to any one of claims 1 to 2, wherein said 1-PEI derivative is PEIm (mannosylated polyethylenimine), preferably in a protonated salt form, wherein the ratio of the protonable N atoms / total N atoms is from 0.1 to 0.8, preferably about 0.4.

4. The pharmaceutical composition according to any one of claims 1 to 3, wherein said sugar alcohol has the general formula (I):
H(HCHO)nH (I)
wherein n is an integer from 2 to 12, and said sugar alcohols include all enantiomers and epimers, and wherein said sugar alcohol is preferably selected from the group consisting of glycerol, mannitol, sorbitol, xylitol and combinations thereof, more preferably said sugar alcohol is D/L-mannitol.

5. The pharmaceutical composition according to any one of claims 1 to 4, wherein said solvent is a pH buffer (6.5 ≤ pH ≤ 8.1), with a weaker base than the polymer, having the general formula (II):
NRR'R" (II)
wherein
if R=R'=R", then R is selected independently from C1-4 alkyl, C1-4 hydroxy-alkyl, C1-5 alkyloxy-C1-4 alkyl;
if R=phenyl; then R',R" is selected independently from C1-4 alkyl, C1-4 hydroxyl-alkyl, C1-5 alkyloxy-C1-4 alkyl, more preferably said solvent is triethylamine or triethanolamine.

6. The pharmaceutical composition according to any one of claims 1 to 5, wherein said macromolecule comprises at least one nucleic acid molecule encoding (poly)peptides that form virus-like particles (VLP).

7. The pharmaceutical composition according to any one of claims 1 to 6, which has enhanced stability at 0-40 °C compared to compositions made without sugar alcohol.

8. The pharmaceutical composition according to any one of claims 1 to 7, wherein the relevant biological activity of the macromolecule is maintained for a longer time period compared to compositions made without sugar alcohol.

9. The pharmaceutical composition according to any one of claims 1 to 8, wherein said macromolecule is a plasmid DNA, and wherein the physico-chemical properties (characterizable by the oc/ccc ratio) of said plasmid DNA are maintained at 0-40 °C for a longer time period compared to compositions made without sugar alcohol.

10. The pharmaceutical formulation according to any one of claims 1 to 10, in lyophilized form.

11. A pharmaceutical composition according to any one of claims 1 to 10, for use in inducing immune responses, for the prevention or treatment of chronic infectious diseases caused by intracellular viruses and bacteria, neoplastic diseases and allergy to be introduced by injection or by mucosal, oral, vaginal, rectal or transdermal administration.

12. Method for the preparation of a pharmaceutical composition comprising nanoparticles with a diameter within the range from 50 to 500 nm being composed of macromolecules and 1-PEI or 1-PEI derivative, said method comprising mixing macromolecules with 1-PEI or 1-PEI derivative in a pharmaceutically acceptable solvent containing a sugar alcohol,
wherein the molar ratio of the 1-PEI or 1-PEI derivative and the macromolecule is from 2 to 10, preferably from 2 to 6, and more preferably from 3 to 4,5, and
wherein the concentration of said sugar alcohols is in the range from 0 to 50% (w/v), preferably from 10% to 30%, and
wherein the ratio of the volume of solvent and the volume of macromolecule solution is from 0 to 20, preferably about 6.

13. The method according to claim 12, further comprising the lypohilization of the composition.

14. The method according to any one of claims 12 or 13, wherein said pharmaceutical- composition is prepared according to any step selected from the group consisting of:
- said macromolecule is reconstituted from a lyophilized state with water;
- said macromolecule is reconstituted from frozen state after thawing;
- said macromolecule and said 1-PEI or 1-PEI derivative are separately frozen in solution, and mixed together after thawing;
- said macromolecule and said 1-PEI or 1-PEI derivative are separately stored in solution, and mixed together;
- said macromolecule is reconstituted from a lyophilized state with a 1-PEI or 1-PEI derivative solution.

15. The pharmaceutical composition or method according to any one of claims 1 to 14, further comprising pharmaceutically acceptable excipients and/or additives.
